Europäisches Patentamt

European Patent Office

Office européen des brevets

(19)

(11) Numéro de publication: **0 373 058**
**A1**

## (12) DEMANDE DE BREVET EUROPEEN

(21) Numéro de dépôt: **89403350.5**

(22) Date de dépôt: **04.12.89**

(51) Int. Cl.⁵: **G01N 33/36, G01N 15/08**

(30) Priorité: **06.12.88 FR 8815949**

(43) Date de publication de la demande:
**13.06.90 Bulletin 90/24**

(84) Etats contractants désignés:
**BE CH DE ES GB IT LI**

(71) Demandeur: **PEIGNAGE AMEDEE**
**154, rue de Cartigny**
**F-59100 Roubaix(FR)**

(72) Inventeur: **Leman,Bernard**
**37 avenue Anatole France**
**F-59100 Roubaix(FR)**
Inventeur: **Delètre,Eric**
**Résidence du Parc 6062 avenue Jean Jaurès**
**F-59100 Roubaix(FR)**

(74) Mandataire: **Lhuillier, René et al**
**ARMENGAUD JEUNE CABINET LEPEUDRY 6,**
**rue du Fg. St-Honoré**
**F-75008 Paris(FR)**

(54) **Dispositif de mesure de la finesse de la laine.**

(57) La mesure est assurée de façon automatique par un appareil muni d'au moins un microordinateur (13) qui recueille non seulement les informations reçues d'un transmetteur de débit (8) d'une pompe à vide (7) et d'un transmetteur de pression différentielle (11), mais aussi les information reçues en permanence d'organes de saisie de facteurs ambiants donnés par un capteur de température ambiante (16), un capteur d'humidité (17) et un capteur de pression atmosphérique (18).

Application à la mesure de la finesse des fibres au cours du traitement de la laine brute.

EP 0 373 058 A1

## DISPOSITIF DE MESURE DE LA FINESSE DE LA LAINE

L'invention concerne un dispositif de mesure automatique de la finesse de la laine, qui compense les variations d'humidité, de pression atmosphérique et de température de l'atmosphère ambiante.

Au cours du traitement de la laine brute, après les opérations de lavage, d'essorage et de cardage, il est nécessaire de pouvoir mesurer la finesse de la laine dont on sait que les fibres peuvent avoir une taille variant de 15 à 45 $\mu$. Il est important en effet de déterminer en permanence cette taille pour savoir dans quelle catégorie ranger le peigné obtenu.

On connaît un certain nombre de méthodes pour déterminer la finesse des fibres, qui remplacent les techniques manuelles et visuelles bien connues consistant à tirer sur les fibres et apprécier à vue leur dimension. La méthode dite "lanamétrique" consiste à effectuer une mesure de finesse individuelle d'une fibre prélevée dans un ensemble, à l'aide d'un microscope muni d'une échelle micrométrique, ou en variante automatisée avec une caméra vidéo associée à un système de reconnaissance et de mesure d'image. Comme il peut y avoir d'assez grosses variations dans le diamètre des fibres, pour pouvoir tirer de ces mesures un enseignement valable, il importe d'effectuer un grand nombre de ces mesures individuelles qui sont longues et soumises aux imprécisions des lectures.

On préfère utiliser maintenant des méthodes basées sur la mesure de la finesse moyenne d'un paquet de fibres à l'aide d'un appareil du type "airflow", dans lequel on fait passer un courant d'air au travers d'une masse de fibres, répartie dans une chambre à fond perforé, connectée à un débitmètre et à un manomètre. Un courant d'air réglé passe à travers les fibres tassées; la différence de pression d'air entre l'entrée et la sortie est fonction de la finesse des fibres, et on lit le diamètre moyen des fibres sur une échelle placée sur le manomètre ou sur le débitmètre. Avec cet appareil de type connu, il est néanmoins nécessaire de tenir compte de l'atmosphère de conditionnement et de mesure du produit. Si les mesures ne sont pas effectuées en atmosphère standard de laboratoire, il faut conditionner l'échantillon de laboratoire à l'équilibre auprès de l'appareil et noter l'humidité relative de l'atmosphère au moment de la mesure puis corriger le résultat final par un facteur de correction. Si l'humidité change pendant la mesure, il faut en tenir compte en effectuant des vérifications de l'humidité au cours d'une série de mesures. Cela revient donc à réétalonner l'appareil au détriment de la précision et de la rapidité d'obtention des résultats. En outre l'interprétation de la lecture visuelle est variable selon l'utilisation à cause des erreurs de parallaxe plus ou moins sensibles selon les personnes. Là encore il faut réétalonner à chaque changement d'utilisateur.

Ces inconvénients ont conduit la demanderesse à rechercher une solution qui, en éliminant ces inconvénients, vise à obtenir un appareil de mesure automatique disposant d'organes de saisie des différents facteurs ambiants notamment humidité et température, appareil dont le réétalonnage est automatique dès que la mesure permanente des facteurs en question indique une variation sensible à partir d'une valeur prédéterminée.

Aussi un objet de l'invention consiste en un dispositif de mesure de la finesse de la laine comportant une cuve à fond perforé connectée à un débitmètre et un manomètre, dans laquelle on dispose un paquet de fibres à mesurer et dans laquelle on fait passer un courant d'air, la différence de pression entre l'entrée et la sortie permettant de déterminer la finesse, dispositif selon lequel la mesure est assurée de façon automatique par un appareil muni d'au moins un microordinateur qui recueille non seulement les informations reçues d'un transmetteur de débit d'une pompe à vide et d'un transmetteur de pression différentielle, mais aussi les informations reçues en permanence d'organes de saisie de divers facteurs ambiants de telle sorte que lorsque lesdits facteurs restent dans des limites acceptables prédéterminées les mesures s'effectuent normalement, mais qu'à l'inverse les mesures sont interrompues ce qui entraîne un réétalonnage automatique de l'appareil.

Selon une caractéristique particulière de l'invention, le transmetteur de débit est branché sur un conduit reliant la partie inférieure de la cuve sous le fond perforé avec la pompe à vide et le transmetteur de pression différentielle est branché sur un autre conduit prenant sur la partie inférieure de la cuve et muni d'une prise de pression atmosphérique.

En outre selon une caractéristique principale de l'invention, les organes de saisie des facteurs ambiants sont constitués d'au moins un capteur de température ambiante, un capteur d'humidité et un capteur de pression atmosphérique.

D'autres caractéristiques particulières et avantages de l'invention apparaîtront à la lecture de la description qui va suivre d'un exemple non limitatif de réalisation faisant référence au dessin annexé qui représente schématiquement l'appareil mettant en oeuvre le dispositif.

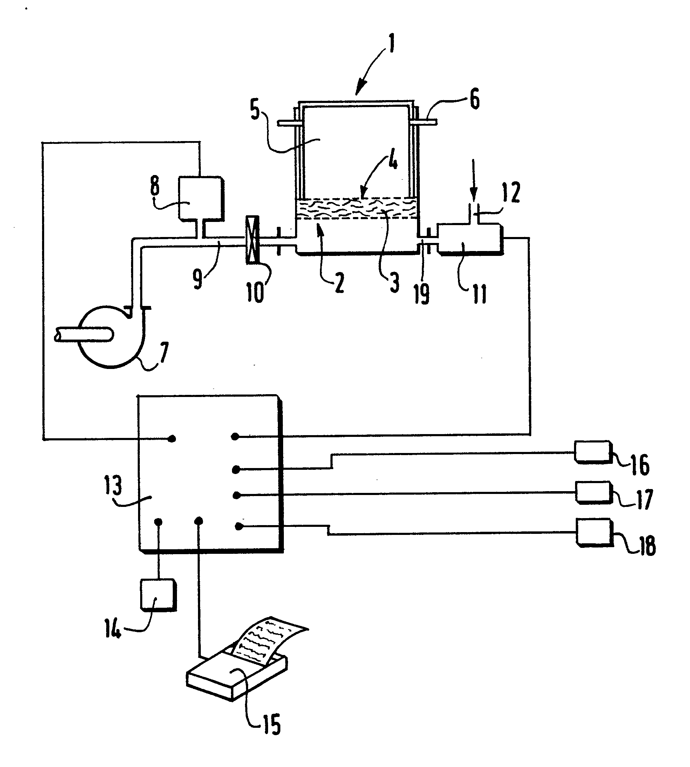

La représentation schématique de la figure 1 montre une cuve de mesure 1 dont la partie inférieure est pourvue d'une tôle perforée 2 au-dessus de laquelle est disposé un paquet de fibres 3 à mesurer. Une seconde tôle perforée 4 constituant le fond d'un bouchon obturateur 5 qui est rapporté dans la cuve, maintient les fibres légèrement tassées entre les deux séries de perforations. Des clés de fermeture 6 permettent le calage de ce bouchon.

La partie de la cuve, sous la tôle perforée 2 est raccordée par un conduit 9 d'un côté à une pompe à vide 7 dont le débit est régulé à partir d'un transmetteur de débit 8; un filtre 10 est intercalé sur le conduit 9 entre la cuve et la prise du transmetteur de débit. Cette même partie inférieure de la cuve est raccordée de l'autre côté par un conduit 19 à un transmetteur de pression différentielle 11 mesurée par capteur piézo électrique de grande précision muni d'une prise 12 de pression atmosphérique. Le transmetteur de débit 8 et le transmetteur de pression différentielle 11 sont connectés à un microordinateur 13 à microprocesseur, disposant d'un afficheur 14 et d'une imprimante 15, microordinateur qui est lui-même connecté à un ordinateur central pour la saisie des résultats. Enfin le microordinateur recueille les informations d'un capteur de température ambiante 16, d'un capteur d'humidité 17 et d'un capteur de pression atmosphérique 18.

On utilise avantageusement cet appareil automatique de la façon suivante. On dispose un paquet de fibres 3 à mesurer dans la cuve 1 au-dessus de la tôle perforée et on place le bouchon 5. La pompe à vide est mise en route et le transmetteur de débit 8 qui capte en permanence le débit dans le conduit 9 fournit une information continue au microordinateur qui permet de réguler automatiquement et en continu ledit débit à une valeur affichée et étalonnée. De l'autre côté le transmetteur de pression différentielle 11 transforme le signal mécanique en signal électronique enregistré dans le microordinateur 13. Ainsi on obtient, du fait de la dépression d'air à débit constant appliquée sous la tôle 2, une information sur la différence de la pression de l'air entre son entrée dans la cuve et la sortie, différence qui est fonction de la finesse des fibres 3. On effectue par exemple trois mesures successives en retournant l'échantillon entre chacune, cela pour obtenir une valeur moyenne de finesse mesurée. Les trois facteurs ambiants, température, humidité et pression atmosphérique, sont mesurés en permanence par les capteurs 16, 17 et 18. Au niveau du microordinateur si l'une ou l'autre des valeurs de ces facteurs dépasse une fourchette de valeurs de consigne prédéterminées et mémorisées, la mesure n'est pas validée et un message d'erreur est affiché sur l'afficheur 14 du type à cristaux liquides, qui indique quel facteur a subi une modification notable. Il convient alors de réétalonner l'appareil, processus qui est engagé automatiquement.

Le système est aussi connectable à tout ordinateur personnel.

## Revendications

1. Dispositif de mesure de la finesse de la laine comportant une cuve à fond perforé connectée à un débitmètre et un manomètre, dans laquelle on dispose un paquet de fibres à mesurer et dans laquelle on fait passer un courant d'air, la différence de pression entre l'entrée et la sortie permettant de déterminer la finesse, caractérisé en ce que la mesure est assurée de façon automatique par un appareil muni d'au moins un microordinateur 13 qui recueille non seulement les informations reçues d'un transmetteur de débit (8) d'une pompe à vide (7) et d'un transmetteur de pression différentielle (11), mais aussi les informations reçues en permanence d'organes de saisie (16, 17, 18) de divers facteurs ambiants de telle sorte que lorsque lesdits facteurs restent dans des limites acceptables prédéterminées les mesures s'effectuent normalement, mais qu'à l'inverse les mesures sont interrompues ce qui entraîne un réétalonnage automatique de l'appareil.

2. Dispositif de mesure selon la revendication 1, caractérisé en ce que le transmetteur de débit (8) est branché sur un conduit (9) reliant la partie inférieure de la cuve (1) sous le fond perforé (2), avec la pompe à vide (7).

3. Dispositif de mesure selon la revendication 1, caractérisé en ce que le transmetteur de pression différentielle (11) est branché sur un autre conduit (19) prenant sur la partie inférieure de la cuve (1) et muni d'une prise (12) de pression atmosphérique.

4. Dispositif de mesure selon la revendication 1, caractérisé en ce que les organes de saisie des facteurs ambiants sont constitués d'au moins un capteur de température ambiante (16), un capteur d'humidité (17) et un capteur de pression atmosphérique (18).

5. Dispositif de mesure selon la revendication 1, caractérisé en ce qu'un afficheur 14 est connecté au microordinateur (13) pour l'affichage, notamment, des valeurs des facteurs ambiants provenant des organes de saisie (16, 17, 18), et de leurs anomalies par rapport à des valeurs de consigne mémorisées.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | US-A-3 039 293  (Reddick et al) * Col. 4, lines 17-67; fig. 3,6 * | 1 | G 01 N   33/36 G 01 N   15/08 |
| A | US-A-2 909 920  (EMMONS) * Col. 2, lignes 40-51; col. 4, lignes 27-50; fig. 3 * | 1 | |
| A | US-A-2 919 573  (BERKLEY et al.) * Col. 2, lignes 16-32; Rev. 1; fig 4,5 * | 1 | |

| DOMAINES TECHNIQUES RECHERCHES (Int. Cl.5) |
|---|
| G 01 N |

Le présent rapport a été établi pour toutes les revendications

| Lieu de la recherche | Date d'achèvement de la recherche | Examinateur |
|---|---|---|
| LA HAYE | 23-01-1990 | KOUZELIS D. |